# EUROPÄISCHE PATENTANMELDUNG

(11) **EP 4 488 374 A1**
(43) Veröffentlichungstag der Anmeldung: **08.01.2025**
(21) Anmeldenummer: 23184204.8
(22) Anmeldetag: 07.07.2023
(51) Int. Cl.: C12P 7/18, C12P 7/52, C12P 7/56, C12N 9/04, C12N 9/88

(54) **VERFAHREN ZUR HERSTELLUNG EINER WÄSSERIGEN LÖSUNG ENTHALTEND ETHYLENGLYCOL**

(71) Anmelder: Annikki GmbH, 8074 Raaba-Grambach (AT)
(72) Erfinder: Staunig, Nicole, 8074 Raaba-Grambach (AT); Frühwirt, Philipp, 8074 Raaba-Grambach (AT)
(74) Vertreter: Schwarz & Partner Patentanwälte GmbH

(57) **Zusammenfassung**

Verfahren zur Herstellung einer wässerigen Lösung enthaltend Ethylenglycol und/oder ein Salz der Brenztraubensäure, indem 2-Keto-3-deoxy-D-xylonat (KDX) und/oder 2-Keto-3-deoxy-L-arabonat (KDA) *in vitro* mit einer Aldolase (EC 4.1.2.55) behandelt wird, wodurch eine wässerige Lösung erhalten wird, welche Glycolaldehyd und ein Salz der Brenztraubensäure enthält, wonach der Glycolaldehyd reduziert wird mittels einer NAD(P)H-abhängigen Alkoholdehydrogenase (EC 1.1.1.1) oder einer NAD(P)H-abhängigen Xylose-Reduktase (EC 1.1.1.307) und anschließend gegebenenfalls entweder das Salz der Brenztraubensäure oder das Ethylenglycol aus der Lösung entfernt werden.

## Beschreibung

Die Erfindung betrifft ein Verfahren zur Herstellung einer wässrigen Lösung, welche Ethylenglycol und ein Salz der Brenztraubensäure (Pyruvat) oder Ethylenglycol und ein Salz der Milchsäure (Lactat) enthält.

### Hintergrund der Erfindung

Ethylenglycol (Ethan-1,2-diol), der einfachste zweiwertige Alkohol, ist eine wichtige Industriechemikalie - es wird z.B. als Frostschutzmittel oder als Precursor für die Polymere Polyethylenterephthalat (PET) und Polyethylenfuranoat (PEF), einer biobasierten Alternative von PET, eingesetzt (Salusjärvi et al., 2019).

Ethylenglycol wird durch die Hydrolyse von Ethylenoxid (bei 200 °C), welches wiederum durch die Oxidation von Ethen (gewonnen aus fossilen Rohstoffen) erhalten wird, hergestellt. Dabei entstehende Nebenprodukte (Oligoethylenglycole) müssen destillativ und damit energieintensiv abgetrennt werden. Zur Vermeidung von Nebenprodukten kann beispielsweise der OMEGA-Prozess (Only Mono Ethylene Glycol Advantage) von Shell eingesetzt werden, bei dem Ethylenoxid zuerst mit CO₂ zu Ethylencarbonat umgesetzt wird, welches unter Freisetzung von CO₂ zu Ethylenglycol hydrolysiert wird (mit KI und K₂M_{O}O₄ als Katalysatoren). Andere Verfahren sind die Rh- oder Co-katalysierte Hydrohydroxymethylierung von Formaldehyd oder die Hydroformylierung von Formaldehyd zu Glycolaldehyd, welcher anschließend zu Ethylenglycol reduziert wird (Berger, 2016).

Da die derzeitigen großtechnischen Verfahren aufgrund des großen Energieeinsatzes (hohe Temperaturen) sowie des fossilen Ursprungs der Rohstoffe (Ethen) oder die Verwendung von teuren, teils toxischen Katalysatoren nicht als nachhaltig bezeichnet werden können, werden und wurden biotechnologische Verfahren entwickelt, die auf nachwachsenden Rohstoffen basieren und bei milderen Reaktionsbedingungen durchgeführt werden.

Um nachhaltige Produktionsverfahren für Chemikalien aus Biomasse zu entwickeln, werden weniger energieintensive Methoden benötigt, welche die bestehende Vielfalt des Biomaterials nutzen. So werden neben effizienten, sanften Methoden zur Depolymerisation von nachwachsenden Rohstoffen zu kürzerkettigen Zuckern effiziente Technologien benötigt, um diese Kohlenhydrat-Intermediate weiter in chemische Produkte umzuwandeln. Eine besondere Herausforderung besteht dabei darin, die nach der Depolymerisation entstehenden Stoffgemische effizient und möglichst vollständig in wertbringende Stoffströme umzuwandeln. Generell enthalten Strohe, Hölzer und andere pflanzenbasierte Rohstoffe, Hemicellulosen und Cellulosen, aus welchen Monomer-Zucker in wechselnder Zusammensetzung freigesetzt werden können, vornehmlich die Pentosen D-Xylose und L-Arabinose, die Hexosen D-Glucose, D-Mannose und D-Galactose sowie von den Zuckern abgeleitete Säuren (z.B. D-Glucuronsäure). Methoden zur effizienten Trennung der C₆- und C₅-Zucker erlauben es, diese Stoffstromtypen getrennt voneinander weiter zu behandeln (z.B. US 9970038 B2). In Mais-Hüllen beispielsweise findet man, bezogen auf den Gesamtzucker, 10% L-Arabinose, 16% D-Xylose, 64% D-Glucose, 4% D-Galactose und 2% D-Mannose (Hromádková & Ebringerovä, 1995), in Weizenstroh hingegen sind es 5% L-Arabinose, 30% D-Xylose, 56% D-Glucose, 1% D-Galactose und 2% D-Mannose (Collins et al., 2014). Diese Prozentwerte zeigen, dass sich die Verhältnisse von L-Arabinose und D-Xylose in beiden Biomassen zueinander stark unterscheiden (Mais-Hüllen: 1:1,6 bzw. Weizenstroh: 1:6).

Derzeit fokussieren biotechnologische Bemühungen vor allem auf Verfahren, die auf die Umwandlung von Biomasse zu chemischen Produkten mittels Fermentationen abzielen, also auf die Stoffumwandlung während des Wachstums von Mikroben in einem Reaktor. Diese fermentativen Ganzzellprozesse sind trotz allen technischen Fortschritts auf dem Gebiet, wie etwa Metabolic Engineering und heterologe Pathway-Expression, durch die physiologischen Limitationen der zellulären Produktion (Toleranz gegenüber Lösungsmitteln, Temperatur, Stofftransport sowie gegenüber hohen Substrat- und Produktkonzentrationen, aber auch durch Nebenprodukte aus anderen Stoffwechselwegen in der Zelle) begrenzt (Claassens et al., 2019). Diese intrazellulären Prozesse und insbesondere der Energiebedarf für die metabolischen (oft auch CO₂-emittierenden) Schritte führen zum einen zu langen Prozesszeiten und zum anderen dazu, dass die erzielbare Kohlenstoffausbeute deutlich unter der theoretisch möglichen liegt. Daneben verwenden diese Prozesse generell meist biosynthetische Stoffwechselwege, die aus dem Substrat CO₂ freisetzen, sodass selbst die theoretische Kohlenstoffausbeute im Produkt nur ein Bruchteil des eingesetzten Kohlenstoffs ist: Während bei Cs-Zuckern je nach Stoffwechselweg 1 - 4 Kohlenstoffe verloren gehen, sind es bei C₅-Zuckern 1 - 3. Typische Eckdaten solcher Prozesse sind in der Literatur zusammengefasst (Salusjärvi et al., 2019).

Alkim et al. beschreiben ein Verfahren zur Herstellung von Ethylenglycol aus D-Xylose über einen synthetischen Stoffwechselweg in *Escherichia coli.* D-Xylose wird zuerst mittels D-Xylose-Isomerase zu D-Xylulose isomerisiert, welche anschließend unter Einsatz von Adenosintriphosphat (ATP) mittels D-Xylulose-1-kinase zu D-Xylulose-1-phosphat phosphoryliert wird. Durch die von der D-Xylulose-1-phosphat-Aldolase katalysierte Aldol-Spaltung werden Dihydroxyacetonphosphat (DHAP) und Glycolaldehyd erhalten, welcher letztlich zum Ethylenglycol reduziert wird (mittels Glycolaldehyd-Reduktase *FucO*)*.* In einem Bioreaktor-Versuch konnten 55 g/l D-Xylose zu 20 g/l Ethylenglycol (91% Ausbeute und 0,37 g/(l·h) Produktivität) umgesetzt werden (Alkim et al., 2015).

Der von Alkim et al. benutzte Stoffwechselweg existiert in nativer Form in *Saccharomyces cerevisiae,* wie Uranukul et al. zeigen konnten. Die beteiligten Enzyme sind die Phosphofructokinase (katalysiert die Phosphorylierung von D-Xylulose) und die Fructose-bisphosphat-Aldolase (katalysiert die Aldol-Spaltung), die Schlüsselenzyme der Glykolyse. Ein Stamm konnte in 330 h 4 g/l Ethylenglycol ausgehend von 50 g/l D-Xylose produzieren, wobei zusätzlich kontinuierlich D-Glucose zugeführt wurde (Uranukul et al., 2018).

Pereira et al. nutzten modifizierte *E*. coli-Zellen zur Umwandlung von D-Xylose und L-Arabinose zu Ethylenglycol. Über mehrere Schritte (Isomerisierung, Epimerisierung und Phosphorylierung) wird D-Xylose in D-Ribulose-1-phosphat bzw. L-Arabinose in L-Xylulose-1-phosphat umgewandelt, welche mittels L-Fuculose-phosphat-Aldolase bzw. L-Rhamnose-l-phosphat-Aldolase in DHAP und Glycolaldehyd gespaltet wird. Auf diese Weise konnte eine Mischung von 15 g/l D-Xylose und 15 g/l L-Arabinose in 220 h zu 10,5 g/l Ethylenglycol umgesetzt werden. Die Daten zeigen, dass D-Xylose schneller zu Ethylenglycol umgesetzt wird und im Falle von L-Arabinose sowie der Pentose-Mischung eine längere Lag-Phase auftritt (Pereira et al., 2016).

Glycolaldehyd, die Vorstufe zu Ethylenglycol, tritt auch als Produkt in einem weiteren Stoffwechselweg ausgehend von D-Xylose oder L-Arabinose auf, der unter dem Namen *Dahms Pathway* (Dahms & Anderson, 1969; Dahms, 1974) bekannt ist. Dabei wird D-Xylose/L-Arabinose zuerst zu 1,4-D-Xylonolacton/1,4-L-Arabinolacton oxidiert, welches unter Einwirkung einer Lactonase zum D-Xylonat/L-Arabonat geöffnet wird. Eine Dehydratase wandelt D-Xylonat/L-Arabonat zu 2-Keto-3-deoxy-D-xylonat (KDX)/2-Keto-3-deoxy-L-arabonat (KDA) um, wobei beide 2-Keto-3-deoxy-lntermediate durch eine Aldolase in Pyruvat und Glycolaldehyd gespaltet werden. Die Verwendung promiskuitiver Enzyme in z.B. *Sulfolobus solfataricus* erlaubt den gleichzeitigen Umsatz von D-Xylose und L-Arabinose (Kopp et al., 2020).

Salusjärvi et al. exprimierten Enzyme des Dahms-Weges (D-Xylose-Dehydrogenase und D-Xylonat-Dehydratase aus *Caulobacter crescentus* sowie eine Aldolase aus *E. coli*) in *Saccharomyces cerevisiae,* wobei neben der Glycolsäure (durch Oxidation mittels einer Aldehyd-Dehydrogenase aus *E. coli*) auch geringe Mengen an Ethylenglycol (durch Reduktion mittels einer endogenen Aldo-Keto-Reduktase) gefunden wurden. Ein Teil der Intermediate wurde von S. *cerevisiae* in Nebenprodukte wie 3-Deoxypentansäure oder 3,4-Dihydroxybutansäure umgewandelt (Salusjärvi et al., 2017).

Chae et al. konstruierten den Dahms-Weg in *E. coli* Stämmen, wobei zur Reduktion von Glycolaldehyd zu Ethylenglycol die Glycolaldehyd-Reduktase *yqhD* verwendet wurde. Unter Fed-Batch-Bedingungen konnten 108,2 g/l Ethylenglycol in einem D-Xylose-Minimalmedium hergestellt werden (Ausbeute 0,36 g/g D-Xylose und 2,25 g/(l·h) Produktivität) (Chae et al., 2018). Wang et al. nutzten einen ähnlichen Zugang, wobei zur Reduktion auf die NADH-abhängige Aldehyd-Reduktase *FucO* statt der NADPHabhängigen Aldehyd-Reduktase *yqhD* verwendet wurde. Dadurch konnten 72 g/l Ethylenglycol aus D-Xylose unter Fed-Batch-Bedingungen (52 h Laufzeit) hergestellt werden (Wang et al., 2018).

Zhang et al. identifizierten den Dahms-Weg auch in dem Mikroorganismus *Enterobacter cloacae* S1. In einer Fed-Batch-Kultur von *E. cloacae* S1 konnten gleichzeitig 34 g/l Ethylenglycol und 13 g/l Glycolsäure in 46 h ausgehend von D-Xylonsäure (0,99 mol Produkte/mol D-Xylonsäure) hergestellt werden (Zhang et al., 2020).

WO 2014162063 A1 beschreibt modifizierte S. *cerevisiae* Stämme, die in der Lage sind, bis zu 1,25 g/l Ethylenglycol aus 20 g/l D-Xylose in 120 h (unter Zusatz von 10 g/l D-Glucose und 10 g/l CaCO₃) über den Dahms-Weg herzustellen.

US 10774348 B2 beschreibt rekombinante Mikroorganismen (*E. coli, S. cerevisiae)* zur Biosynthese von Ethylenglycol in Kombination mit einer C3-Verbindung wie Aceton, 2-Propanol oder Propen (ausgehend von Pyruvat als Produkt des Dahms-Weges oder von DHAP als Spalt-Produkt von D-Ribulose-1-phosphat oder D-Xylulose-1-phosphat). So werden beispielsweise von einem *E*. coli-Stamm 15 g/l D-Xylose innerhalb von 48 h zu 5 g/l Ethylenglycol, 0,17 g/l Aceton und 0,42 g/l 2-Propanol über den Dahms-Weg umgewandelt.

Pyruvat, das zweite Produkt des Dahms-Weges, ist ein zentrales Intermediat im zellulären Stoffwechsel und dient beispielsweise als Vorstufe zur Aminosäure Alanin oder zum Acetyl-CoA, welches am Citratzyklus beteiligt ist. Durch Reduktion von Pyruvat gelangt man zu Milchsäure (Lactat), welche über einen breiten Anwendungsbereich verfügt. Milchsäure wird z.B. als Säuerungsmittel in der Lebensmittelindustrie, als Entkalkungsmittel, pH-Regulator oder Reinigungsmittel in der chemischen Industrie, als Zusatz in Anti-Akne-Cremes oder Feuchtigkeitscremes in der kosmetischen Industrie sowie als Precursor für Acrylsäure oder Ethyllactat verwendet (Wee et al., 2006). Darüber hinaus kann Milchsäure auch zum Hautpeeling *(Chemisches Peeling)* eingesetzt werden (Smith, 1996). Die bifunktionelle Milchsäure lässt sich des Weiteren auch polymerisieren, wobei Polylactid (PLA), ein bioabbaubarer und biokompatibler Kunststoff, entsteht, der Anwendungen in der Verpackungsindustrie, Textilindustrie, im Elektronik- sowie im Medizinbereich hat (Balla et al., 2021).

Jia et al. entwickelten eine enzymatische Kaskade zur Herstellung von (R)-Acetoin ((R)-3-Hydroxy-2-butanon) und Ethylenglycol ausgehend von D-Xylose über den Dahms-Weg. Die enzymatische Reduktion von Glycolaldehyd und Ethylenglycol treibt dabei die Oxidation der D-Xylose zu D-Xylonolacton an (in situ-Regeneration des Kofaktors NAD). 2 Pyruvat-Moleküle werden schließlich mittels α-Acetolactat-Synthase und α-Acetolactat-Decarboxylase unter zweifacher Abspaltung von CO₂ zu (R)-Acetoin umgewandelt. Auf diese Weise können 10 mM (1,5 g/l) D-Xylose nach 24 h zu 5,5 mM Ethylenglycol sowie 3,2 mM (R)-Acetoin umgesetzt werden (Jia et al., 2018).

Boer et al. präsentierten eine *in vitro* Variante des Dahms-Weges mit gereinigten Enzymisolaten, bei der D-Xylose bzw. D-Xylonolacton (1 mM Substrat-Konzentration) zu Ethylenglycol (Reduktion von Glycolaldehyd), Glycolsäure (Oxidation von Glycolaldehyd) oder Lactat (Reduktion von Pyruvat) umgewandelt wird. Hauptaugenmerk der Studie war die Anwendung eines spektrophotometrischen Assays zur Evaluierung der Effizienz der Kaskade, die auf der Bildung bzw. dem Verbrauch von NADH basiert. Dazu wurden 2 mM NAD externer Kofaktor zugesetzt. Weiters wurde in der Studie auch die Rolle der Lactonase (aus *Caulobacter crescentus)* im Dahms-Weg untersucht. Die Daten legen nahe, dass die spontane Ringöffnung des D-Xylonolactons vor allem bei pH 7 geschwindigkeitsbestimmend ist. Erst durch die Zugabe der Lactonase wird die Gesamt-Kinetik der Kaskade merklich beschleunigt, was aber auch durch die Erhöhung des pH-Wertes erreicht werden kann (Boer et al., 2019). Insgesamt beschreiben Boer et al. kein Verfahren, bei welchem die genannten Chemikalien in einem präparativen Maßstab hergestellt werden können. So ist z.B. die Konzentration der Reaktionspartner sehr klein, z.B. ca. 0,3 mM im Falle des Ethylenglycols.

Hier setzt nun die vorliegende Erfindung an und setzt sich zum Ziel, ein Verfahren zur Herstellung einer wässerigen Lösung enthaltend Ethylenglycol und ein Salz der Milchsäure (Lactat) oder Ethylenglycol und ein Salz der Brenztraubensäure (Pyruvat) zur Verfügung zu stellen, das von 2-Keto-3-deoxy-D-xylonat (KDX) und/oder 2-Keto-3-deoxy-L-arabonat (KDA) ausgeht, bei höheren Konzentrationen technisch durchführbar ist und außerdem hohe Ausbeuten ermöglicht.

### Detaillierte Beschreibung der Erfindung

Die Aufgabe der Erfindung zur Herstellung einer wässerigen Lösung enthaltend Ethylenglycol und/oder ein Salz der Brenztraubensäure wird gelöst, indem 2-Keto-3-deoxy-D-xylonat (KDX) und/oder 2-Keto-3-deoxy-L-arabonat (KDA) *in vitro* mit einer Aldolase behandelt wird, wodurch eine wässerige Lösung erhalten wird, welche Glycolaldehyd und ein Salz der Brenztraubensäure enthält, wonach der Glycolaldehyd durch Behandeln mit einer NAD(P)H-abhängigen Alkoholdehydrogenase oder mit einer NAD(P)H-abhängigen Xylose-Reduktase *in vitro* zu Ethylenglycol reduziert wird, wobei der durch die Reduktion mit der Alkoholdehydrogenase oder zusammen mit der Xylose-Reduktase entstandene oxidierte Kofaktor NAD(P)⁺ mittels eines sekundären Alkohols unter Bildung eines Ketons reduziert wird und anschließend gegebenenfalls entweder das Salz der Brenztraubensäure oder das Ethylenglycol aus der Lösung entfernt werden.

Die Aufgabe der Erfindung zur Herstellung einer wässerigen Lösung enthaltend Ethylenglycol und/oder ein Salz der Milchsäure wird gelöst, indem 2-Keto-3-deoxy-D-xylonat (KDX) und/oder 2-Keto-3-deoxy-L-arabonat (KDA) *in vitro* mit einer Aldolase behandelt wird, wodurch eine wässerige Lösung erhalten wird, welche Glycolaldehyd und ein Salz der Brenztraubensäure enthält, wonach der Glycolaldehyd durch Behandeln mit einer NAD(P)H-abhängigen Alkoholdehydrogenase *in vitro* zu Ethylenglycol und das Salz der Brenztraubensäure *in vitro* mittels einer Lactat-Dehydrogenase zu einem Salz der Milchsäure reduziert wird, wobei der durch die Reduktion mit der Alkoholdehydrogenase entstandene oxidierte Kofaktor NAD(P)⁺ mittels eines sekundären Alkohols unter Bildung eines Ketons reduziert wird und anschließend gegebenenfalls entweder das Salz der Milchsäure oder das Ethylenglycol aus der Lösung entfernt werden.

Die Erfindung betrifft ferner ein Verfahren zur Herstellung einer wässerigen Lösung enthaltend Ethylenglycol, indem Glycolaldehyd, welcher in einer wässerigen Lösung gelöst vorliegt, durch Behandeln mit einer NAD(P)H-abhängigen Alkoholdehydrogenase oder zusammen mit einer NAD(P)H-abhängigen Xylose-Reduktase *in vitro* zu Ethylenglycol reduziert und der durch die Reduktion entstandene oxidierte Kofaktor NAD(P)⁺ mittels eines sekundären Alkohols unter Bildung eines Ketons reduziert wird.

In den erfindungsgemäßen Verfahren wird als der sekundäre Alkohol bevorzugt 2-Propanol eingesetzt.

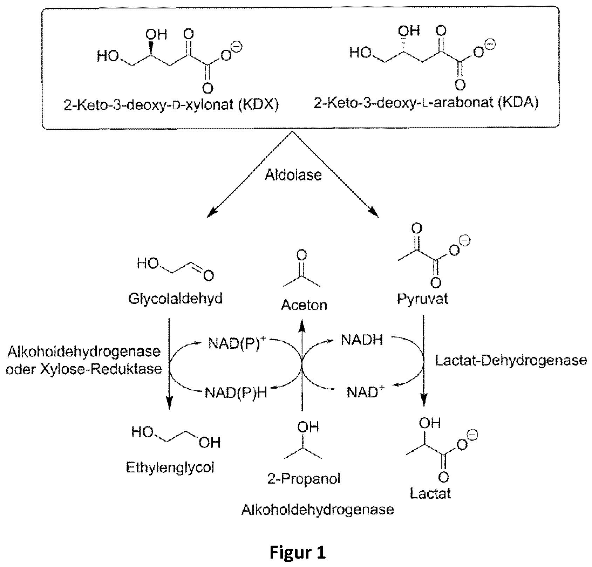

Die erfindungsgemäßen Verfahren sind in der beiliegenden Figur 1 schematisch dargestellt.

Es hat sich überaschenderweise gezeigt, dass die erfindungsgemäß gesetzten Ziele erreicht werden können, wenn der durch die Reduktion mit der Alkoholdehydrogenase oder zusammen mit der Xylose-Reduktase entstandene oxidierte Kofaktor NAD(P)⁺ mittels eines sekundären Alkohols, vorzugsweise 2-Propanol, unter Bildung eines Ketons reduziert wird.

Ferner ist es entscheidend, dass die Umsetzung nicht fermentativ durchgeführt wird, sondern die Enzyme als solche in der wässerigen Lösung enthalten sind, also *in vitro* gearbeitet wird.

Die Regeneration des Kofaktors mittels Alkoholdehydrogenase ist z.B. aus der EP 2812439 B1 vorbekannt oder von Xu et al. (2021) beschrieben.

Eine weitere bevorzugte Variante des erfindungsgemäßen Verfahrens ist dadurch gekennzeichnet, dass es als Eintopf-Reaktion ohne Isolierung von Zwischenprodukten durchgeführt wird.

In weiteren bevorzugten Ausführungsformen des erfindungsgemäßen Verfahrens beträgt die Konzentration von KDA und/oder KDX in der wässerigen Lösung 20 - 300 g/l.

Der besonders bevorzugte Temperaturbereich liegt zwischen 25 und 40 °C.

Der besonders bevorzugte pH-Bereich der Reaktion liegt zwischen 7,0 und 8,5.

Es hat sich ferner gezeigt, dass die in den erfindungsgemäßen Verfahren die Enzyme am besten als Lysat der entsprechenden, sie bildenden Zellen vorliegen.

In einer weiteren Variante können die Enzyme als Suspension, im Homogenat vorliegen oder auch auf einem festen Trägermaterial immobilisiert sein.

Suspension bedeutet in diesem Kontext eine Suspension von *resting cells.* Diese werden nach der Kultivierung geerntet (vom Nährmedium abgetrennt) und in einem geeigneten Puffersystem suspendiert. Im Gegensatz zu fermentativen Verfahren, bei denen auch mit ganzen Zellen gearbeitet wird, können die *resting cells* aufgrund des nicht Vorhandensein von Kohlenstoff-Quellen und Nährstoffen nicht mehr wachsen, sondern dienen nur der Umsetzung von Substraten (Lin & Tao, 2017). Homogenat steht in diesem Kontext für eine physikalisch und/oder chemisch behandelte Suspension (z.B. mittels Druckes, Lysozym oder Ultraschall behandelt), wobei die Zellbestandteile aus den Zellen freigesetzt werden. Ein Lysat wird erhalten, wenn die unlöslichen Zellbestandteile des Homogenats beispielsweise durch Filtration oder Zentrifugation entfernt werden (siehe *Produktion der Enzyme & Herstellung der Lysate* für Details).

Die im Verfahren verwendete Aldolase zur Spaltung von KDX und KDA in Glycolaldehyd und Pyruvat kann aus einer der Gruppen EC 4.1.2.18 (2-Dehydro-3-deoxy-L-pentonat-Aldolase), 4.1.2.20 (2-Dehydro-3-deoxyglucarat-Aldolase), 4.1.2.21 (2-Dehydro-3-deoxy-6-phosphogalactonat-Aldolase), 4.1.2.28 (2-Dehydro-3-deoxy-D-pentonat-Aldolase), 4.1.2.29 (5-Dehydro-2-deoxyphosphogluconat-Aldolase), 4.1.2.51 (2-Dehydro-3-deoxy-D-gluconat-Aldolase), 4.1.2.52 (4-Hydroxy-2-oxoheptandioat-Aldolase), 4.1.2.53 (2-Keto-3-deoxy-L-rhamnonat-Aldolase), 4.1.2.54 (L-*threo*-3-Deoxy-hexylosonat-Aldolase), 4.1.2.55 (2-Dehydro-3-deoxy-phosphogluconat/2-Dehydro-3-deoxy-6-phosphogalactonat-Aldolase) sowie 4.1.3.39 (4-Hydroxy-2-oxovalerat-Aldolase) stammen, wobei die Gruppe 4.1.2.55 (2-Dehydro-3-deoxy-phosphogluconat/2-Dehydro-3-deoxy-6-phosphogalactonat-Aldolase) besonders bevorzugt ist.

Die zur Reduktion von Pyruvat zu Lactat verwendeten Oxidoreduktase stammt vorzugsweise aus der Gruppe EC 1.1.1.27 (L-Lactat-Dehydrogenase).

Bei der zur Reduktion von Glycolaldehyd zu Ethylenglycol eingesetzten Oxidoreduktase handelt es sich bevorzugt um eine Alkoholdehydrogenase oder eine Xylose-Reduktase.

Die NAD(P)H-abhängige Alkoholdehydrogenase zur Reduktion von Glycolaldehyd zu Ethylenglycol umfasst vorzugsweise eine Aminosäuresequenz, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet, wobei stringente Bedingungen vorzugsweise einen oder mehrere Waschschritte bei 65 °C und einer Salzkonzentration von 0,1x bis 2x SSC umfassen.

Die NAD(P)H-abhängige Xylose-Reduktase zur Reduktion von Glycolaldehyd zu Ethylenglycol umfasst vorzugsweise eine Aminosäuresequenz, welche ausgewählt ist aus der Gruppe bestehend aus:
i) einer Aminosäuresequenz, die eine Identität zu SEQ ID Nr. 7 von mindestens 80% aufweist,
ii) einer Aminosäuresequenz, die durch eine Nukleinsäure kodiert wird, die eine Identität zu SEQ ID Nr. 8 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 bindet, wobei stringente Bedingungen vorzugsweise einen oder mehrere Waschschritte bei 65 °C und einer Salzkonzentration von 0,1x bis 2x SSC umfassen.

SEQ ID Nr. 1: 
MKAAVVEQFKKPLQVKEVEKPKISYGEVLVRIKACGVCHTDLHAAHGDWPVKPKLPLIPGHEGVGVIEEVGPGV
THLKVGDRVGIPWLYSACGHCDYCLSGQETLCERQQNAGYSVDGGYAEYCRAAADYVVKIPDNLSFEEAAPIFC
AGVTTYKALKVTGAKPGEWVAIYGIGGLGHVAVQYAKAMGLNVVAVDLGDEKLELAKQLGADLVVNPKHDD
AAQWIKEKVGGVHATVVTAVSKAAFESAYKSIRRGGACVLVGLPPEEIPIPIFDTVLNGVKIIGSIVGTRKDLQEAL
QFAAEGKVKTIVEVQPLENINDVFDRMLKGQINGRVVLKVD
SEQ ID Nr. 2: 
ATGAAAGCTGCAGTTGTGGAACAATTTAAAAAGCCGTTACAAGTGAAAGAAGTGGAAAAACCTAAGATCT
CATACGGGGAAGTATTAGTGCGCATCAAAGCGTGTGGGGTATGCCATACAGACTTGCATGCCGCACATGG
CGACTGGCCTGTAAAGCCTAAACTGCCTCTCATTCCTGGCCATGAAGGCGTCGGTGTAATTGAAGAAGTAG
 
GTCCTGGGGTAACACATTTAAAAGTTGGAGATCGCGTAGGTATCCCTTGGCTTTATTCGGCGTGCGGTCAT
TGTGACTATTGCTTAAGCGGACAAGAAACATTATGCGAACGTCAACAAAACGCTGGCTATTCCGTCGATGG
TGGTTATGCTGAATATTGCCGTGCTGCAGCCGATTATGTCGTAAAAATTCCTGATAACTTATCGTTTGAAGA
AGCCGCTCCAATCTTTTGCGCTGGTGTAACAACATATAAAGCGCTCAAAGTAACAGGCGCAAAACCAGGTG
AATGGGTAGCCATTTACGGTATCGGCGGGCTTGGACATGTCGCAGTCCAATACGCAAAGGCGATGGGGTT
AAACGTCGTTGCTGTCGATTTAGGTGATGAAAAACTTGAGCTTGCTAAACAACTTGGTGCAGATCTTGTCG
TCAATCCGAAACATGATGATGCAGCACAATGGATAAAAGAAAAAGTGGGCGGTGTGCATGCGACTGTCGT
CACAGCTGTTTCAAAAGCCGCGTTCGAATCAGCCTACAAATCCATTCGTCGCGGTGGTGCTTGCGTACTCGT
CGGATTACCGCCGGAAGAAATACCTATTCCAATTTTCGATACAGTATTAAATGGAGTAAAAATTATTGGTTC
TATCGTTGGTACGCGCAAAGACTTACAAGAGGCACTTCAATTTGCAGCAGAAGGAAAAGTAAAAACAATT
GTCGAAGTGCAACCGCTTGAAAACATTAACGACGTATTCGATCGTATGTTAAAAGGGCAAATTAACGGCCG
CGTCGTGTTAAAAGTAGATTAA
SEQ ID Nr. 3: 
MSGKMKAAVVHEFGKPLTIEELDIPTIKPTQILVKMIACGVCHTDLHAASGDWPKKPHLPFIPGHEGVGTVVQV
GSEVDWVKEGDVVGVPWLYSACGHCEHCLAGWETLCAKQEETGYSVNGCFAEYVVADPNYIAHLPKGVDPV
KVAPVLCAGLTVYKGLKMTDTRAGNWVAISGVGGLGQMAVQYAVAMGLNVVAVDIDDEKLATAKKLGATYT
VNARNTDPAAFMQEKVGGVHGGLITAVSTKAFSQAMGYVRAGGTLVLNGLPPGDFPISIFDVVMNAITIRGSI
VGTRLDMIEALSFFAEGKVTSVTTTDRIDNINAIFDALKNGRVEGRVVLDFRN
SEQ ID Nr. 4: 
ATGTCCGGAAAAATGAAAGCCGCTGTGGTTCATGAATTTGGCAAACCACTGACCATTGAAGAACTGGACAT
TCCCACCATTAAACCCACCCAGATTTTGGTTAAAATGATTGCCTGCGGCGTGTGCCATACAGATTTGCATGC
TGCCAGCGGGGATTGGCCCAAAAAACCACATCTGCCGTTTATTCCCGGCCACGAAGGCGTTGGCACGGTTG
TGCAGGTTGGCAGCGAAGTAGACTGGGTAAAGGAAGGCGACGTTGTTGGCGTGCCTTGGCTCTATTCTGC
CTGCGGCCATTGCGAGCATTGTCTGGCTGGGTGGGAAACCCTTTGTGCGAAGCAAGAAGAAACCGGCTAT
TCCGTAAACGGCTGCTTTGCCGAATACGTGGTAGCAGACCCTAACTACATTGCTCATCTTCCCAAAGGCGTA
GACCCTGTAAAAGTTGCCCCCGTACTGTGCGCAGGCCTGACCGTTTATAAGGGCCTGAAAATGACAGACAC
GCGGGCAGGGAACTGGGTTGCCATTTCCGGCGTTGGGGGGTTAGGCCAGATGGCTGTGCAGTACGCCGT
AGCAATGGGGCTGAATGTTGTGGCGGTAGATATTGATGATGAAAAACTGGCAACCGCCAAAAAGCTTGGT
GCAACGTACACTGTTAACGCCAGAAATACAGATCCGGCAGCATTTATGCAGGAAAAAGTTGGTGGCGTAC
ACGGTGGATTGATCACCGCTGTTTCCACCAAAGCATTTTCTCAGGCTATGGGTTATGTGCGGGCAGGGGGC
ACTTTGGTGCTAAATGGGCTGCCGCCGGGTGATTTCCCAATTTCTATCTTTGACGTGGTCATGAATGCCATT
ACCATTCGCGGCTCCATAGTAGGCACACGGCTGGACATGATTGAAGCTCTTTCCTTCTTTGCGGAAGGGAA
 
GGTAACATCTGTCACCACAACGGATCGGATTGATAACATCAACGCAATTTTTGATGCGCTCAAGAACGGTC
GGGTGGAAGGCCGCGTGGTTCTGGACTTCCGCAACTGA
SEQ ID Nr. 5: 
MNKTMKAAVARAFGKPLEIEEVEVPRPRAGELLVKIEACGVCHTDLHAVEGDWPVKPNPPFIPGHEGVGHVV
AVGEGVTHVKEGDRVGIPWLYSACGHCEHCLGGWETLCEQQQNAGYSVNGGFAEYALAAADYVGLLPKNVG
FVDIAPVLCAGVTVYKGLKMTDTRPGNWVVVSGIGGLGHMAVQYARAMGLNVAAVDIDDDKLDFAKRLGAE
VVVNAKATDPAAYLKKEIGGAHGALITAVSPKAFEQALGMVRRGGTVALNGLPPGDFPLSIFDMVLNGVTVRG
SIVGSRLDLQESLQFAEEGKVRATVATEKLENINSVFDRMRRGQIEGRIVLDMAA
SEQ ID Nr. 6: 
ATGAACAAGACCATGAAGGCCGCGGTGGCCCGCGCGTTCGGCAAACCCCTGGAAATCGAAGAAGTCGAG
GTGCCGCGCCCGCGCGCGGGCGAACTGTTGGTGAAGATCGAGGCCTGCGGCGTCTGCCACACCGATCTGC
ACGCGGTCGAGGGCGACTGGCCGGTCAAGCCCAATCCCCCTTTCATCCCGGGCCACGAAGGCGTGGGCCA
CGTGGTGGCGGTGGGCGAAGGCGTGACGCACGTCAAGGAAGGCGACCGCGTCGGCATCCCCTGGCTGTA
TTCCGCCTGTGGCCATTGCGAGCATTGCCTGGGCGGCTGGGAGACGCTGTGCGAACAGCAGCAGAACGCC
GGCTACTCCGTCAACGGCGGCTTCGCCGAGTACGCGCTGGCCGCGGCCGACTACGTGGGCCTGCTGCCGA
AGAATGTCGGCTTCGTCGACATCGCCCCGGTACTGTGCGCCGGCGTCACGGTCTACAAAGGCCTGAAGAT
GACGGACACACGGCCAGGCAACTGGGTCGTGGTCTCCGGCATCGGCGGCCTGGGCCACATGGCGGTGCA
GTATGCGCGCGCCATGGGCTTGAACGTGGCGGCCGTGGACATCGACGACAAACTGGATTTCGCCAAG
CGTCTCGGCGCCGAGGTGGTCGTGAATGCCAAGGCCACCGATCCAGCGGCTTACCTGAAGAAGGAAATCG
GCGGCGCCCACGGCGCGCTGATCACGGCGGTTTCGCCCAAGGCCTTCGAGCAGGCGCTGGGCATGGTGC
GTCGCGGCGGCACGGTGGCGCTCAACGGCCTGCCGCCGGGCGACTTCCCGCTGTCGATCTTCGACATGGT
GCTCAACGGCGTGACGGTGCGCGGTTCGATCGTGGGTTCGCGCCTGGACTTGCAGGAATCGCTGCAGTTC
GCCGAGGAAGGCAAGGTGCGCGCGACCGTGGCGACCGAGAAGCTGGAGAACATCAACAGCGTGTTCGAC
CGCATGCGCCGCGGCCAGATCGAAGGCCGCATCGTGCTGGACATGGCGGCGTAA
SEQ ID Nr. 7: 
MSTTVNTPTIKLNSGYEMPLVGFGCWKVTNATAADQIYNAIKTGYRLFDGAEDYGNEKEVGEGINRAIKDGLVK
REELFITSKLWNNFHDPKNVETALNKTLSDLNLDYVDLFLIHFPIAFKFVPIEEKYPPGFYCGDGDNFHYEDVPLLD
TWKALEKLVEAGKIKSIGISNFTGALIYDLIRGATIKPAVLQIEHHPYLQQPKLIEYVQKAGIAITGYSSFGPQSFLEL
ESKRALNTPTLFEHETIKSIADKHGKSPAQVLLRWATQRNIAVIPKSNNPERLAQNLSVVDFDLTKDDLDNIAKLD
IGLRFNDPWDWDNIPIFV
SEQ ID Nr. 8:

ATGAGCACGACCGTTAATACTCCGACGATCAAACTGAACTCCGGTTACGAGATGCCGCTGGTTGGCTTTGG
CTGTTGGAAAGTTACCAATGCGACGGCGGACCAGATTTATAACGCTATTAAAACCGGTTACCGTCTGT
TCGATGGCGCAGAGGACTACGGTAACGAGAAAGAAGTGGGTGAAGGTATTAATCGCGCAATTAAAGACG
GTCTGGTCAAACGTGAAGAGCTGTTTATTACCTCGAAACTGTGGAATAACTTCCATGATCCTAAGAACGTG
GAGACTGCGCTGAATAAGACCCTGAGCGATCTGAACCTGGACTATGTGGATCTGTTTCTGATTCACTTCCC
GATTGCTTTCAAATTTGTCCCGATCGAAGAAAAGTACCCACCGGGCTTCTACTGCGGTGACGGCGACAACT
TCCATTATGAGGATGTTCCGCTGCTGGACACGTGGAAGGCGCTGGAGAAATTGGTGGAAGCCGGTAAGAT
CAAGTCCATTGGCATTAGCAACTTCACCGGTGCCTTGATTTACGATTTGATCCGTGGTGCGACCATTAAACC
GGCGGTTCTGCAGATCGAGCATCACCCGTATCTGCAACAGCCGAAACTGATCGAATACGTTCAGAAAGCA
GGTATCGCCATCACTGGCTATAGCAGCTTCGGTCCACAGAGCTTCCTGGAGCTGGAGAGCAAGCGTGCCCT
GAATACCCCGACGTTGTTTGAACACGAAACCATCAAGTCTATCGCTGACAAACACGGTAAGAGCCCTGCAC
AAGTCCTGCTGCGCTGGGCAACGCAACGTAATATTGCGGTTATTCCGAAGAGCAATAACCCGGAGCGTCTG
GCGCAGAATCTGTCTGTCGTCGACTTTGATTTGACCAAGGATGACCTGGATAATATCGCGAAGCTGGACAT
CGGCCTGCGCTTCAACGATCCGTGGGATTGGGACAACATCCCGATCTTTGTGTA

Die NAD(P)H-abhängige Alkoholdehydrogenase zur Reduktion von Glycolaldehyd zu Ethylenglycol umfasst oder besteht vorzugsweise aus einer Aminosäuresequenz, die eine Identität zu SEQ ID Nr. 1, 3 oder 5 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße NAD(P)H-abhängige Alkoholdehydrogenase zur Reduktion von Glycolaldehyd zu Ethylenglycol die Aminosäuresequenz SEQ ID Nr. 1, 3 oder 5 oder besteht aus dieser.

Alternativ umfasst oder besteht die NAD(P)H-abhängige Alkoholdehydrogenase zur Reduktion von Glycolaldehyd zu Ethylenglycol vorzugsweise aus einer Aminosäuresequenz, die durch eine Nukleinsäure kodiert wird, die eine Identität zu SEQ ID Nr. 2, 4 oder 6 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße NAD(P)H-abhängige Alkoholdehydrogenase zur Reduktion von Glycolaldehyd zu Ethylenglycol kodiert, die Nukleinsäuresequenz SEQ ID Nr. 2, 4 oder 6 oder besteht aus dieser.

Die NAD(P)H-abhängige Xylose-Reduktase zur Reduktion von Glycolaldehyd zu Ethylenglycol umfasst oder besteht vorzugsweise aus einer Aminosäuresequenz, die eine Identität zu SEQ ID Nr. 7 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die erfindungsgemäße NAD(P)H-abhängige Xylose-Reduktase zur Reduktion von Glycolaldehyd zu Ethylenglycol die Aminosäuresequenz SEQ ID Nr. 7 oder besteht aus dieser.

Alternativ umfasst oder besteht die NAD(P)H-abhängige Xylose-Reduktase zur Reduktion von Glycolaldehyd zu Ethylenglycol vorzugsweise aus einer Aminosäuresequenz, die durch eine Nukleinsäure kodiert wird, die eine Identität zu SEQ ID Nr. 8 von mindestens 80%, noch mehr bevorzugt von 85%, noch mehr bevorzugt von 90%, noch mehr bevorzugt von 95%, noch mehr bevorzugt von 98%, noch mehr bevorzugt von 99%, insbesondere von 100%, aufweist. Besonders bevorzugt umfasst die Nukleinsäure, welche die erfindungsgemäße NAD(P)H-abhängige Xylose-Reduktase zur Reduktion von Glycolaldehyd zu Ethylenglycol kodiert, die Nukleinsäuresequenz SEQ ID Nr. 8 oder besteht aus dieser.

Der Begriff "Identität", wie hier verwendet, bezieht sich auf den Prozentsatz der identischen Nukleotid- bzw. Aminosäureübereinstimmungen zwischen mindestens zwei, unter Verwendung eines standardisierten Algorithmus miteinander ausgerichteten Nukleotid- bzw. Aminosäuresequenzen ("Alignment"). Ein solcher Algorithmus kann, in einer standardisierten und reproduzierbaren Weise, Lücken ("Gap") in die verglichenen Sequenzen einfügen, um das Alignment zwischen zwei Sequenzen zu optimieren, und somit einen aussagekräftigeren Vergleich der beiden Sequenzen erreichen.

Die prozentuale Identität zwischen Sequenzen kann unter Verwendung von ein oder mehreren Computeralgorithmen oder im Stand der Technik bekannten oder hierin beschriebenen Programmen bestimmt werden. Erfindungsgemäß wird zur Bestimmung der Identität das durch National Center for Biotechnology Information (NCBI) bereitgestellte Basic Local Alignment Search Tool (BLAST) (Altschul et al., 1990), verwendet. Die BLAST-Software-Reihe enthält verschiedene Programme, einschließlich ein als "BLAST 2 Sequences" genanntes Werkzeug, das für den direkten paarweisen Vergleich von zwei Nukleotid- bzw. Aminosäuresequenzen verwendet wird. "BLAST 2 Sequences" kann auch über die NCBI World Wide Web-Seite im Internet interaktiv abgerufen und verwendet werden. Das blastn-Programm (für Nukleotidsequenzen) verwendet als Vorgaben eine Wortlänge (W) von 28, eine Erwartung (E) von 0,05, M = 1, N = -2 und einen Vergleich beider Stränge. Für Aminosäuresequenzen verwendet das blastp-Programm als Vorgaben eine Wortlänge von 3 und eine Erwartung (E) von 0,05 und die BLOSUM62-Scoring-Matrix (Henikoff & Henikoff, 1989), Alignments (B) von 50, Erwartung (E) von 0,05, M = 1, N = - 2.

Alternativ umfasst die NAD(P)H-abhängige Alkoholdehydrogenase zur Reduktion von Glycolaldehyd zu Ethylenglycol vorzugsweise eine Aminosäuresequenz oder besteht aus dieser, die durch eine Nukleinsäure kodiert wird, die unter stringenten Bedingungen an ein Nukleinsäuremolekül mit der Nukleinsäuresequenz SEQ ID Nr. 2, 4 oder 6 bindet. Wie hierin verwendet, beziehen sich die stringenten Bedingungen auf Bedingungen, unter denen sogenannte spezifische Hybride, jedoch keine unspezifischen Hybride gebildet werden.

Beispielsweise kann die Hybridisierung durch herkömmlich bekannte Verfahren durchgeführt werden, wie die von J. Sambrook et al. in Molecular Cloning, A Laboratory Manual, 2nd Ed., Cold Spring Harbor Laboratory (1989) beschriebenen. Unter stringenter Bedingung ist eine Bedingung gemeint, bei der das Waschen bei 65 °C und einer Salzkonzentration von 0,1x bis 2x SSC durchgeführt wird (wobei unter 1xSSC eine Mischung aus 0,15 M Natriumchlorid/0,015 M Natriumcitrat zu verstehen ist).

Die erfindungsgemäß eingesetzten Enzyme zur Reduktion von Glycolaldehyd zu Ethylenglycol sind vorzugsweise NAD(P)H-abhängige Alkoholdehydrogenasen und NAD(P)H-abhängige Xylose-Reduktasen. Es hat sich überraschenderweise gezeigt, dass diese Enzyme in der Lage sind, Glycolaldehyd in Anwesenheit des Kofaktors NAD(P)H zu Ethylenglycol umzusetzen. Dementsprechend betrifft ein weiterer Aspekt der vorliegenden Erfindung die Verwendung dieser Enzyme zur Reduktion von Glycolaldehyd zu Ethylenglycol in Anwesenheit des Kofaktors NAD(P)H.

Vorzugsweise betrifft ein Aspekt der vorliegenden Erfindung die Verwendung einer NAD(P)H-abhängigen Alkoholdehydrogenase und/oder einer NAD(P)H-abhängigen Xylose-Reduktase zur Reduktion von Glycolaldehyd zu Ethylenglycol, wobei die NAD(P)H-abhängige Alkoholdehydrogenase vorzugsweise eine Aminosäuresequenz umfasst, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet, wobei stringente Bedingungen vorzugsweise einen oder mehrere Waschschritte bei 65 °C und einer Salzkonzentration von 0,1x bis 2x SSC umfassen,
und die NAD(P)H-abhängige Xylose-Reduktase vorzugsweise eine Aminosäuresequenz umfasst, welche ausgewählt ist aus der Gruppe bestehend aus:
i) einer Aminosäuresequenz, die eine Identität zu SEQ ID Nr. 7 von mindestens 80% aufweist,
ii) einer Aminosäuresequenz, die durch eine Nukleinsäure kodiert wird, die eine Identität zu SEQ ID Nr. 8 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 bindet, wobei stringente Bedingungen vorzugsweise einen oder mehrere Waschschritte bei 65 °C und einer Salzkonzentration von 0,1x bis 2x SSC umfassen.

Mit den nachfolgenden Beispielen werden bevorzugte Ausführungsformen der Erfindung noch näher beschrieben.

### Materialien

Natrium-Pyruvat, Natrium-L-Lactat und Glycolaldehyd-Dimer, Ethylenglycol sowie IPTG (Isopropyl-β-D-thiogalactopyranosid) wurden von Sigma-Aldrich, Magnesiumchlorid-Hexahydrat, Kaliumdihydrogenphosphat, di-Kaliumhydrogenphosphat sowie Natriumdodecylsulfat (SDS) wurden von Carl Roth, NAD⁺, NADH-Dinatriumsalz, NADP⁺-Dinatriumsalz, NADPH-Tetranatriumsalz, und Methanol wurden von PanReac AppliChem (ITW Reagents) und Triethanolamin wurde von Chem-Lab NV bezogen.

Lösungen von 2-Keto-3-deoxy-L-arabonat (KDA) bzw. 2-Keto-3-deoxy-D-xylonat (KDX) wurden enzymatisch mittels L-Arabonat-Dehydratase (*Azotobacter vinelandii* DJ; NCBI Database Number: ACO81022.1) aus Kalium-L-Arabonat bzw. Kalium-D-Xylonat hergestellt (Weimberg, 1959), welche durch chemische Oxidation von L-Arabinose bzw. D-Xylose erhalten wurden (Moore & Link, 1940).

### Produktion der Enzyme & Herstellung der Lysate

### Allgemeines zur Expression von rekombinanten Enzymen in E. coli

Für die rekombinante Enzymproduktion in einem *Escherichia* coli-Stamm wurde zunächst das zu exprimierende Gen in einer PCR unter der Verwendung der genomischen DNA oder deren synthetisch an die Codon-Verwendung von *E. coli* angepasstes Äquivalent als Matrize zusammen mit spezifischen Oligonukleotiden, die zusätzlich Erkennungssequenzen für Restriktionsendonukleasen tragen, amplifiziert und aus dem Reaktionsgemisch isoliert. Nach dem Nukleinsäure-Verdau mit den Restriktionsenzymen Sphl und Hindlll wurde das für das Target-Enzym kodierende Genfragment in das mit Sphl und Hindlll geschnittene Rückgrat des Expressionsvektors pQE70-Kan ligiert. Das Ligationsprodukt wurde in chemisch kompetente *E*. coli-Zellen Top10F transformiert und die entstandenen Kolonien wurden für die Plasmid-Isolierung und Restriktionsanalyse benutzt.

Das Ergebnis des Klonierungs-Schritts wurde mittels Restriktionsenzym-Verdau und DNA-Sequenzierung überprüft. Das resultierende Konstrukt trägt das Target-Gen unter dem IPTG-induzierbaren T5-Promotor.

Für die Überexpression des Enzymes in *E. coli* wurde das resultierende Expressionsplasmid in die kompetenten Expressionszellen RB791 transformiert. Nach 24 h Inkubation bei 37 °C wurden entstandene Kolonien für die Expressionstests in LB-Medium angeimpft.

Am nächsten Tag wurden damit Expressionskulturen mit einer optische Dichte OD₅₅₀ von 0,02 angeimpft und bei 37 °C geschüttelt, bis eine OD₅₅₀ von 0,3 erreicht wurde. Anschließend wurde die Temperatur auf 25 °C gesenkt und die Kulturen wurden beim Erreichen einer OD₅₅₀ von 0,5 mit 0,1 mM IPTG induziert. Nach 22 h wurden die Kulturen geerntet (vom Medium mittels Zentrifugation in Form eines Zellpellets abgetrennt) und auf die Expression des rekombinanten Enzymes mit Hilfe von SDS-Gelelektrophorese und einer Aktivitätsbestimmung (Verwendung in Use-Test oder optisch-enzymatischer Assay) analysiert.

### Herstellung von Zell-Lysaten mittels GEA-Aufschlusses

Zur Herstellung einer Zell-Suspension (Volumen > 50 ml) wurde das nach obigen Verfahren hergestellte Zellpellet in einem geeigneten Gefäß eingewogen und in Puffer (Triethanolamin (TEA) - HCl oder Kaliumphosphat-Puffer (KPP); siehe Tabelle 1 für verwendete Puffer-Systeme) gelöst. Der Massenanteil an Biomasse beträgt üblicherweise 20%, der Rest entfällt auf den Puffer.

Zum Zell-Aufschluss wurde ein Lab Homogenizer PandaPLUS 2000 von GEA verwendet (Druck ca. 1000 bar).

Das Homogenat wurde 10 min lang bei 4 °C und 16000 rpm zentrifugiert (Hermle Z 446 K Zentrifuge), um die unlöslichen Zellfragmente abzutrennen und das Lysat zu erhalten.

### Herstellung von Zell-Lysaten mittels Sonifier-Aufschlusses

Zur Herstellung einer Zell-Suspension wurde das nach obigen Verfahren hergestellte Zellpellet in einem geeigneten Gefäß eingewogen und mit Puffer und Lysozym (finale Konzentration 0,5 mg/ml) versetzt (siehe Tabelle 1 für verwendete Puffer-Systeme) und unter Rührung gelöst. Der Massenanteil an Biomasse beträgt üblicherweise 20%, der Rest entfällt auf den Puffer.

Zum Zell-Aufschluss wurde ein Branson Sonifier 450 verwendet. Die Suspension wurde dreimal mit je 15 Ultraschall-Stößen (Einstellungen am Gerät: Timer = 15; Duty Cycle = 50; Output Control = 3 - 5) behandelt.

Das erhaltene Homogenat wurde 10 min lang bei 4 °C und 16000 rpm zentrifugiert (Eppendorf Zentrifuge 5417R), um die unlöslichen Zellfragmente abzutrennen und das Lysat zu erhalten.

**Tabelle 1. Spenderorganismen und Aufschlussbedingungen für die in den Beispielen verwendeten Enzyme.**

| **Enzymtyp (EC-Klasse)** | **katalysierte Reaktion(en)** | **Spenderorganismus** | **Aufschlussbedingungen** | **Literatur** |
|---|---|---|---|---|
| Aldolase (EC 4.1.2.55) | KDX / KDA → Glycolaldehyd + Pyruvat | *Sulfolobus acidocaldarius* | GEA (20% Biomasse in 100 mM TEA-HCl, pH 7) | Wolterink-van Loo et al., 2009) |
| Alkoholdehydrog enase I (EC 1.1.1.1) | Glycolaldehyd → Ethylenglycol; 2-Propanol → Aceton | *Geobacillus stearothermophilus* | GEA (20% Biomasse in 100 mM TEA-HCl, pH 7 + 0,5 mM ZnCl₂) | (NCBI Protein Database: WP_033015595.1); SEQ ID Nr. 1 |
| Alkoholdehydrog enase II (EC 1.1.1.1) | Glycolaldehyd → Ethylenglycol; 2-Propanol --> Aceton | *Acetobacter pasteurianus* | Sonifier (20% Biomasse in 100 mM TEA-HCl) | (NCBI Protein Database: AEN03783.1); SEQ ID Nr. 3 |
| Alkoholdehydrog enase III (EC 1.1.1.1) | Glycolaldehyd → Ethylenglycol; 2-Propanol → Aceton | *Achromobacter xylosoxidans* | Sonifier (20% Biomasse in 100 mM TEA-HCl) | (NCBI Protein Database: WP_006385522.1); SEQ ID Nr. 5 |
| Xylose-Reduktase | Glycolaldehyd → Ethylenglycol | *Candida tropicalis* | GEA (20% Biomasse in 100 mM TEA-HCl) | (NCBI Protein Database: ABX60132.1); SEQ ID Nr. 7 |
| Alkoholdehydrog enase IV (EC 1.1.1.2) | 2-Propanol → Aceton | *Thermoanaerobacter pseudethanolicus* | GEA (20% Biomasse in 100 mM TEA-HCl pH 7 + 0,5 mM ZnCl₂) | (NCBI Protein Database: ABY93890.1) |
| L-Lactat-Dehydrogenase (EC 1.1.1.27) | Pyruvat → L-Lactat | *Oryctolagus cuniculi* (Kaninchenmuskel) | GEA (20% Biomasse in 100 mM TEA-HCl pH 7) | (Zheng et al., 2004) |

### Analytische Methoden

### High Performance Anion Exchange Chromatography (HPAEC)

Substrat-Umsätze und Produkt-Konzentrationen wurden durch HPAEC (High Performance Anion Exchange Chromatography) ermittelt. Dazu wurde ein Dionex ICS6000 System mit AS-AP Autosampler verwendet. Die Messung der organischen Säuren bzw. deren Anionen (2-Keto-3-deoxy-D-xylonat, 2-Keto-3-deoxy-L-arabonat, Lactat und Pyruvat) erfolgte mittels Leitfähigkeitsdetektion (CD) gekoppelt an einen Dionex AERS 500 elektrolytisch regenerierten Suppressor im externen Wassermodus. Zur Trennung der Analyten wurde eine Dionex IonPac AS11-HC-4µm Säule mit entsprechender Vorsäule sowie einem NaOH-Gradienten verwendet. Das Laufmittel wurde zusätzlich mit einer Dionex ATC Anion Trap Column vorbehandelt.

### High Performance Liquid Chromatography (HPLC)

Zur Quantifizierung von Glycolaldehyd und Ethylenglycol wurde HPLC (High Performance Liquid Chromatography) verwendet. Detektion erfolgt mittels eines Brechungsindex-Detektors. Zur Messung wird eine Phenomenex Rezex ROA-Organic Acid H+ (8%) Säule mit entsprechender Vorsäule verwendet und mit 1 mM Schwefelsäure isokratisch eluiert.

### Bestimmung von Enzym-Aktivitäten (optisch-enzymatischer Assay)

Enzym-Aktivitäten in den Lysaten wurden mit einem Shimadzu UV-1900 Spektrophotometer bestimmt. Dazu wurde die Bildung bzw. der Verbrauch von NAD(P)H bei einer Wellenlänge von 340 nm über die Änderung der Absorption verfolgt. Die Messungen wurden mit 0,2 mM Kofaktor (NAD(P)⁺oder NAD(P)H) durchgeführt. Dazu wurden 20 µl einer 10 mM Stock-Lösung des Kofaktors in einer Küvette (Greiner bioone Semi-Micro-Küvette aus Polystyrol) vorgelegt und der gewünschte pH-Wert wurde mit 100 mM TEA-HCl-Puffer eingestellt (870 µl). 10 µl Lysat (verdünnt oder unverdünnt) und 100 µl Substrat-Lösung wurden zur Küvette zugesetzt und die Messung unmittelbar danach gestartet. Die Messungen wurden standardmäßig bei 25 °C durchgeführt. Über den Extinktionskoeffizienten von NADH bzw. NADPH bei 340 nm (*ε* = 6220 L mol⁻¹ cm⁻¹) kann die Enzym-Aktivität des Lysats in U/ml (bezogen auf das Volumen des Lysats) oder U/g (bezogen auf die zur Herstellung eingesetzte Biomasse) bestimmt werden. 1 U steht dabei für 1 µmol Substratumsatz pro Minute (1 U = 1 µmol/min = 1,67·10⁻⁸ kat).

Mit den nachfolgenden Beispielen werden bevorzugte Varianten des erfindungsgemäßen Verfahrens noch näher beschrieben. Die in diesen Beispielen eingesetzten Lysate wurden nach den oben beschriebenen Verfahren hergestellt.

### Beispiel 1

### Reduktion von Glycolaldehyd zu Ethylenglycol

In einem 2 ml Glas-Vial (Ansatz 1) wurden folgende Komponenten vermischt: 50 µl eines 1000 mM TEA-HCl-Puffers (pH 7), 290 µl deionisiertes Wasser, 100 µl einer Glycolaldehyd-Lösung (50 g/l), 10 µl Alkoholdehydrogenase I-Lysat sowie 50 µl 2-Propanol. Der Ansatz wurde 21 h bei 30 °C unter kontinuierlichem Schütteln (1200 rpm, Eppendorf Thermomixer) inkubiert.

In einem 2 ml Glas-Vial (Ansatz 2) wurden folgende Komponenten vermischt: 50 µl eines 1000 mM TEA-HCl-Puffers (pH 7), 275 µl deionisiertes Wasser, 100 µl einer Glycolaldehyd-Lösung (50 g/l), 10 µl Xylose-Reduktase-Lysat, 2U Alkoholdehydrogenase IV-Lysat, 5 µl einer 5 mM NADP⁺-Lösung (final 50 µM) sowie 50 µl 2-Propanol. Der Ansatz wurde 21 h bei 30 °C unter kontinuierlichem Schütteln (1200 rpm, Eppendorf Thermomixer) inkubiert.

Zur Aufarbeitung wurden 100 µl des Ansatzes mit 100 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. 150 µl des klaren Überstands wurden in HPLC-Vials überführt und vermessen (HPLC, RI-Detektion).

In Ansatz 1 wurde Glycolaldehyd (167 mM) vollständig zu Ethylenglycol reduziert. In Ansatz 2 wurden 89% des Glycolaldehyds zu Ethylenglycol (157 mM) reduziert.

### Beispiel 2

### Umwandlung einer 9/1-Mischung von 2-Keto-3-deoxy-D-xylonat (KDX) und 2-Keto-3-deoxy-L-arabonat (KDA) zu Ethylenglycol und Lactat

In einem 2 ml Glas-Vial wurden folgende Komponenten vermischt: 50 µl eines 2000 mM TEA-HCl-Puffers (pH 8,5), 136 µl deionisiertes Wasser, 200 µl einer Lösung aus KDX/KDA (9/1 m/m), 10 µl Aldolase-Lysat, 2U Alkoholdehydrogenase I-Lysat, 2U Lactat-Dehydrogenase-Lysat sowie 50 µl 2-Propanol. Der Ansatz wurde 20 h bei 30 °C unter kontinuierlichem Schütteln (1200 rpm, Eppendorf Thermomixer) inkubiert.

Zur Aufarbeitung wurden 100 µl des Ansatzes mit 100 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. Der klare Überstand wurde 1:250 verdünnt und mittels HPAEC (CD) vermessen. Für die HPLC wurden 150 µl des klaren Überstands in ein HPLC-Vial überführt und vermessen (RI-Detektion).

Auf diese Weise konnten die 2-Keto-3-deoxy-zuckersäuren vollständig zu Lactat (94 mM) und Ethylenglycol (98 mM) umgesetzt werden.

### Beispiel 3

### Umwandlung einer 9/1-Mischung von 2-Keto-3-deoxy-o-xylonat (KDX) und 2-Keto-3-deoxy-L-arabonat (KDA) zu Ethylenglycol und Pyruvat

In einem 2 ml Glas-Vial (Ansatz 1) wurden folgende Komponenten vermischt: 50 µl eines 2000 mM TEA-HCl-Puffers (pH 8,5), 167 µl deionisiertes Wasser, 200 µl einer Lösung aus KDX/KDA (9/1 m/m)*, 10 µl Aldolase-Lysat, 2U Alkoholdehydrogenase I-Lysat sowie 50 µl 2-Propanol. Der Ansatz wurde 20 h bei 30 °C unter kontinuierlichem Schütteln (1200 rpm, Eppendorf Thermomixer) inkubiert.

In einem 2 ml Glas-Vial (Ansatz 2) wurden folgende Komponenten vermischt: 50 µl eines 2000 mM TEA-HCl-Puffers (pH 8,5), 153 µl deionisiertes Wasser, 200 µl einer Lösung aus KDX/KDA (9/1 m/m), 10 µl Aldolase-Lysat, 20 µl Xylose-Reduktase-Lysat, 2U Alkoholdehydrogenase IV-Lysat, 5 µl einer 5 mM NADP⁺-Lösung (final 50 µM) sowie 50 µl 2-Propanol. Der Ansatz wurde 20 h bei 30 °C unter kontinuierlichem Schütteln (1200 rpm, Eppendorf Thermomixer) inkubiert.

Zur Aufarbeitung wurden 100 µl des Ansatzes mit 100 µl Reinstwasser und 200 µl MeOH versetzt und bei 60 °C für 20 min inkubiert (1200 rpm, Eppendorf Thermomixer). Nach der Denaturierung wurde die Probe 10 min bei max. g zentrifugiert. Der klare Überstand wurde 1:250 verdünnt und mittels HPAEC (CD) vermessen. Für die HPLC wurden 150 µl des klaren Überstands in ein HPLC-Vial überführt und vermessen (RI-Detektion).

In Ansatz 1 wurden die 2-Keto-3-deoxy-zuckersäuren vollständig zu Pyruvat (90 mM) und Ethylenglycol (96 mM) umgesetzt. In Ansatz 2 wurden 89% der 2-Keto-3-deoxy-zuckersäuren zu Pyruvat (76 mM) und Ethylenglycol (84 mM) umgesetzt.

### Literatur

Salusjärvi, L., Havukainen, S., Koivistoinen, O., & Toivari, M. (2019). Biotechnological production of glycolic acid and ethylene glycol: current state and perspectives. Applied Microbiology and Biotechnology, 103, 2525-2535. https://doi.org/10.1007/s00253-019-09640-2
Berger, A. (2016). Ethylenglycol, RD-05-01999 in Böckler, F., Dill, B., Eisenbrand, G., Faupel, F., Fugmann, B., Gamse, T., Matissek, R., Pohnert, G., Rühling, A., Schmidt, S., Sprenger, G. (Ed.), RÖMPP [Online], Stuttgart, Georg Thieme Verlag. https://roempp.thieme.de/lexicon/RD-05-01999 (aufgerufen am 30.06.2023)
Hromádková, Z. & Ebringerovä, A. (1995). Isolation and Characterization of Hemicelluloses of Corn Hulls. Chemical Papers 49(2), 97-101. https://chempap.org/?id=7&paper=3556
Collins, S. R. A., Wellner, N., Martinez Bordonado, I., Harper, A. L., Miller, C. N., Bancroft, I., & Waldron, K. W. (2014). Variation in the chemical composition of wheat straw: the role of tissue ratio and composition. Biotechnology for Biofuels, 7, 121. https://doi.org/10.1186/s13068-014-0121-y Claassens, N. J., Burgener, S., Vögeli, B., Erb, T. J., & Bar-Even, A. (2019). A critical comparison of cellular and cell-free bioproduction systems. Current Opinion in Biotechnology, 60, 221-229. https://doi.org/10.1016/j.copbio.2019.05.003
Alkim, C., Cam, Y., Trichez, D., Auriol, C., Spina, L., Vax, E., Bartolo, F., Besse, P., François, J. M., & Walther, T. (2015). Optimization of ethylene glycol production from (D)-xylose via a synthetic pathway implemented in Escherichia coli. Microbial Cell Factories, 14, 127. https://doi.org/10.1186/s12934-015-0312-7
Uranukul, B., Woolston, B. M., Fink, G. R., & Stephanopoulos, G. (2018). Biosynthesis of monoethylene glycol in Saccharomyces cerevisiae utilizing native glycolytic enzymes. Metabolie Engineering, 51, 20-31. https://doi.org/10.1016/j.ymben.2018.09.012
Pereira, B., Li, Z.-J., De Mey, M., Lim, C. G., Zhang, H., Hoeltgen, C., & Stephanopoulos G. (2016). Efficient utilization of pentoses for bioproduction of the renewable two-carbon compounds ethylene glycol and glycolate. Metabolie Engineering, 34, 80-87. https://doi.org/10.1016/j.ymben.2015.12.004
Dahms, A. S., & Anderson, R. L. (1969). 2-Keto-3-deoxy-L-arabonate aldolase and its role in a new pathway of L-arabinose degradation. Biochemical and Biophysical Research Communications, 36(5), 809-814. https://doi.org/10.1016/0006-291X(69)90681-0
Dahms, A. S. (1974). 3-Deoxy-D-pentulosonic acid aldolase and its role in a new pathway of D-xylose degradation. Biochemical and Biophysical Research Communications, 60(4), 1433-1439. https://doi.org/10.1016/0006-291X(74)90358-1
Kopp, D., Bergquist, P. L., & Sunna, A. (2020). Enzymology of Alternative Carbohydrate Catabolic Pathways. Catalysts, 10(11), 1231. https://doi.org/10.3390/catal10111231
Salusjärvi, L., Toivari, M., Vehkomäki, M.-L. Koivistoinen, O., Mojzita, D., Niemelä, K., Penttilä, M. & Ruohonen, L. (2017). Production of ethylene glycol or glycolic acid from D-xylose in Saccharomyces cerevisiae. Applied Microbiology and Biotechnology, 101, 8151-8163. https://doi.org/10.1007/s00253-017-8547-3
Chae, T. U., Choi, S. Y., Ryu, J. Y., & Lee, S. Y. (2018). Production of ethylene glycol from xylose by metabolically engineered Escherichia coli. AIChE Journal, 64(12), 4193-4200. https://doi.org/10.1002/aic.16339
Wang, Y., Xian, M., Feng, X., Liu, M., & Zhao, G. (2018). Biosynthesis of ethylene glycol from D-xylose in recombinant Escherichia coli. Bioengineered, 9, 233-241. https://doi.org/10.1080/21655979.2018.1478489
Zhang, Z., Yang, Y., Wang, Y., Gu, J., Lu, X., Liao, X., Shi, J., Kim, C. H., Lye, G., Baganz, F., & Hao, J. Ethylene glycol and glycolic acid production from xylonic acid by Enterobacter cloacae. Microbial Cell Factories, 19, 89 (2020). https://doi.org/10.1186/s12934-020-01347-8
Wee, Y.-J., Kim, J.-N., & Ryu, H.-W. (2006). Biotechnological Production of Lactic Acid and Its Recent Applications. Food Technology & Biotechnology, 44(2), 163-172. https://www.ftb.com.hr/80-volume-44-issue-no-2/445-biotechnological-production-of-lactic-acid-and-its-recent-applications Smith, W. P. (1996). Epidermal and dermal effects of topical lactic acid. Journal of the American Academy of Dermatology, 35(3), 388-391. https://doi.org/10.1016/s0190-9622(96)90602-7
Balla, E., Daniilidis, V., Karlioti, G., Kalamas, T., Stefanidou, M., Bikiaris, N.D., Vlachopoulos, A., Koumentakou, I., Bikiaris, D. N. (2021). Poly(lactic Acid): A Versatile Biobased Polymer for the Future with Multifunctional Properties-From Monomer Synthesis, Polymerization Techniques and Molecular Weight Increase to PLA Applications. Polymers, 13(11), 1822. https://doi.org/10.3390/polym13111822
Jia, X., Kelly, R. M., & Han, Y. (2018). Simultaneous biosynthesis of (R)-acetoin and ethylene glycol from D-xylose through in vitro metabolic engineering. Metabolie Engineering Communications, 7, e00074. https://doi.org/10.1016/j.mec.2018.e00074
Boer, H., Andberg, M., Pylkkänen, R., Maaheimo, H., and Koivula, A. (2019). In vitro reconstitution and characterisation of the oxidative D-xylose pathway for production of organic acids and alcohols. AMB Express, 9, 48. https://doi.org/10.1186/s13568-019-0768-7
Xu, J., Zhou, H., Yu, H., Deng, T., Wang, Z., Zhang, H., Wu, J., & Yang, L. (2021). Computational design of highly stable and soluble alcohol dehydrogenase for NADPH regeneration. Bioresources and Bioprocessing, 8, 12. https://doi.org/10.1186/s40643-021-00362-w
Lin, B., & Tao, Y. (2017). Whole-cell biocatalysts by design. Microbial Cell Factories, 16, 106. https://doi.org/10.1186/s12934-017-0724-7
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Protein [Internet]. Bethesda (MD): National Library of Medicine (US), National Center for Biotechnology Information. Accession No. ACO81022.1, L-arabonate dehydratase [Azotobacter vinelandii DJ]; [aufgerufen am 05.07.2023]. Verfügbar unter: https://www.ncbi.nlm.nih.gov/protein/226721851
Weimberg, R. (1959). L-2-Keto-4,5-dihydroxyvaleric Acid: an Intermediate in the Oxidation of L-Arabinose by Pseudomonas saccharophila. Journal of Biological Chemistry, 234(4), 727-732. https://doi.org/10.1016/50021-9258(18)70163-4
Moore, S. & Link, K.P. (1940). CARBOHYDRATE CHARACTERIZATION: I. THE OXIDATION OF ALDOSES BY HYPOIODITE IN METHANOL II. THE IDENTIFICATION OF SEVEN ALDO-MONOSACCHARIDES AS BENZIMIDAZOLE DERIVATIVES. Journal of Biological Chemistry, 133, 293-311. https://doi.org/10.1016/S0021-9258(18)73312-7
Wolterink-van Loo, S., Siemerink, M. A. J., Perrakis, G., Kaper, T., Kengen, S. W. M., & van der Oost, J. (2009). Improving low-temperature activity of Sulfolobus acidocaldarius 2-keto-3-deoxygluconate aldolase. Archaea, 2(4), 233-239. https://doi.org/10.1155/2009/194186
Protein [Internet]. Bethesda (MD): National Library of Medicine (US), National Center for Biotechnology Information. Accession No. WP_033015595.1, alcohol dehydrogenase AdhP [Geobacillus stearothermophilus]; [aufgerufen am 29.06.2023]. Verfügbar unter: https:jjwww.ncbi.nlm.nih.govjproteinjWP_033015595.1
Protein [Internet]. Bethesda (MD): National Library of Medicine (US), National Center for Biotechnology Information. Accession No. AEN03783.1, NAD+-dependent and Zn-containing alcohol dehydrogenase [Acetobacter pasteurianus NBRC 101655]; [aufgerufen am 29.06.2023]. Verfügbar unter: https://www.ncbi.nlm.nih.gov/protein/AEN03783.1
Protein [Internet]. Bethesda (MD): National Library of Medicine (US), National Center for Biotechnology Information. Accession No. WP_006385522.1, alcohol dehydrogenase AdhP [Achromobacter xylosoxidans]; [aufgerufen am 29.06.2023]. Verfügbar unter: https:jjwww.ncbi.nlm.nih.govjproteinjWP_006385522.1
Protein [Internet]. Bethesda (MD): National Library of Medicine (US), National Center for Biotechnology Information. Accession No. ABX60132.1, xylose reductase [Candida tropicalis]; [aufgerufen am 29.06.2023]. Verfügbar unter: https://www.ncbi.nlm.nih.gov/protein/ABX60132.1
Protein [Internet]. Bethesda (MD): National Library of Medicine (US), National Center for Biotechnology Information. Accession No. ABY93890.1, Alcohol dehydrogenase, zinc-binding domain protein [Thermoanaerobacter pseudethanolicus ATCC 33223]; [aufgerufen am 30.06.2023]. https://www.ncbi.nlm.nih.gov/protein/ABY93890.1
Zheng, Y., Guo, S., Guo, Z., & Wang, X. (2004). Effects of N-terminal deletion mutation on rabbit muscle lactate dehydrogenase. Biochemistry (Moscow), 69(4), 401-406. https://doi.org/10.1023/b:biry.0000026195.31821.e1

## Patentansprüche

1. Verfahren zur Herstellung einer wässerigen Lösung enthaltend Ethylenglycol und/oder ein Salz der Brenztraubensäure, indem 2-Keto-3-deoxy-D-xylonat (KDX) und/oder 2-Keto-3-deoxy-L-arabonat (KDA) *in vitro* mit einer Aldolase behandelt wird, wodurch eine wässerige Lösung erhalten wird, welche Glycolaldehyd und ein Salz der Brenztraubensäure enthält, wonach der Glycolaldehyd durch Behandeln mit einer NAD(P)H-abhängigen Alkoholdehydrogenase oder mit einer NAD(P)H-abhängigen Xylose-Reduktase *in vitro* zu Ethylenglycol reduziert wird, wobei der durch die Reduktion mit der Alkoholdehydrogenase oder zusammen mit der Xylose-Reduktase entstandene oxidierte Kofaktor NAD(P)⁺ mittels eines sekundären Alkohols unter Bildung eines Ketons reduziert wird und anschließend gegebenenfalls entweder das Salz der Brenztraubensäure oder das Ethylenglycol aus der Lösung entfernt werden.

2. Verfahren zur Herstellung einer wässerigen Lösung enthaltend Ethylenglycol und/oder ein Salz der Milchsäure, indem 2-Keto-3-deoxy-D-xylonat (KDX) und/oder 2-Keto-3-deoxy-L-arabonat (KDA) *in vitro* mit einer Aldolase behandelt wird, wodurch eine wässerige Lösung erhalten wird, welche Glycolaldehyd und ein Salz der Brenztraubensäure enthält, wonach der Glycolaldehyd durch Behandeln mit einer NAD(P)H-abhängigen Alkoholdehydrogenase *in vitro* zu Ethylenglycol und das Salz der Brenztraubensäure *in vitro* mittels einer Lactat-Dehydrogenase zu einem Salz der Milchsäure reduziert wird, wobei der durch die Reduktion mit der Alkoholdehydrogenase entstandene oxidierte Kofaktor NAD(P)⁺ mittels eines sekundären Alkohols unter Bildung eines Ketons reduziert wird und anschließend gegebenenfalls entweder das Salz der Milchsäure oder das Ethylenglycol aus der Lösung entfernt werden.

3. Verfahren zur Herstellung einer wässerigen Lösung enthaltend Ethylenglycol, indem Glycolaldehyd, welcher in einer wässerigen Lösung gelöst vorliegt, durch Behandeln mit einer NAD(P)H-abhängigen Alkoholdehydrogenase oder zusammen mit einer NAD(P)H-abhängigen Xylose-Reduktase *in vitro* zu Ethylenglycol reduziert und der durch die Reduktion entstandene oxidierte Kofaktor NAD(P)⁺ mittels eines sekundären Alkohols unter Bildung eines Ketons reduziert wird.

4. Verfahren nach einem der Ansprüche 1 bis 3, **dadurch gekennzeichnet, dass** der sekundäre Alkohol 2-Propanol ist.

5. Verfahren nach einem der Ansprüche 1 bis 4, **dadurch gekennzeichnet, dass** die Enzyme als Lysat der entsprechenden, sie bildenden Zellen vorliegen.

6. Verfahren nach einem der Ansprüche 1 bis 5, **dadurch gekennzeichnet, dass** die NAD(P)H-abhängige Alkoholdehydrogenase zur Reduktion von Glycolaldehyd zu Ethylenglycol eine Aminosäuresequenz umfasst, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet.

7. Verfahren nach einem der Ansprüche 1 bis 6, **dadurch gekennzeichnet, dass** die NAD(P)H-abhängige Xylose-Reduktase zur Reduktion von Glycolaldehyd zu Ethylenglycol eine Aminosäuresequenz umfasst, welche ausgewählt ist aus der Gruppe bestehend aus:
i) einer Aminosäuresequenz, die eine Identität zu SEQ ID Nr. 7 von mindestens 80% aufweist,
ii) einer Aminosäuresequenz, die durch eine Nukleinsäure kodiert wird, die eine Identität zu SEQ ID Nr. 8 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 bindet.

8. Verwendung einer NAD(P)H-abhängigen Alkoholdehydrogenase und/oder einer NAD(P)H-abhängigen Xylose-Reduktase zur Reduktion von Glycolaldehyd zu Ethylenglycol, wobei die NAD(P)H-abhängige Alkoholdehydrogenase vorzugsweise eine Aminosäuresequenz umfasst, welche ausgewählt ist aus der Gruppe bestehend aus:
i) einer Aminosäuresequenz, die eine Identität zu SEQ ID Nr. 1, 3 oder 5 von mindestens 80% aufweist,
ii) einer Aminosäuresequenz, die durch eine Nukleinsäure kodiert wird, die eine Identität zu SEQ ID Nr. 2, 4 oder 6 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 2, 4 oder 6 bindet,
und die NAD(P)H-abhängige Xylose-Reduktase vorzugsweise eine Aminosäuresequenz umfasst, welche ausgewählt ist aus der Gruppe bestehend aus:
i) einer Aminosäuresequenz, die eine Identität zu SEQ ID Nr. 7 von mindestens 80% aufweist,
ii) einer Aminosäuresequenz, die durch eine Nukleinsäure kodiert wird, die eine Identität zu SEQ ID Nr. 8 von mindestens 80% aufweist, und
iii) einer Aminosäuresequenz, die durch eine Nukleinsäure kodiert wird, die unter stringenten Bedingungen an ein Nukleinsäuremolekül mit der Nukleinsäuresequenz SEQ ID Nr. 8 bindet
